# EUROPEAN PATENT SPECIFICATION

(11) **EP 4 306 948 B1**
(45) Date of publication and mention of the grant of the patent: **26.08.2026**
(21) Application number: 22184288.3
(22) Date of filing: 12.07.2022
(51) Int. Cl.: G01N 27/416, G01N 27/48

(54) **VALIDATION OF ELECTROCHEMICAL MEASUREMENTS USING A POTENTIOSTAT**
VALIDIERUNG VON ELEKTROCHEMISCHEN MESSUNGEN UNTER VERWENDUNG EINES POTENTIOSTATEN
VALIDATION DES MESURES ÉLECTROCHIMIQUES À L'AIDE D'UN POTENTIOSTAT

(43) Date of publication of application: 17.01.2024
(73) Proprietor: Infineon Technologies AG, 85579 Neubiberg (DE)
(72) Inventor: SIEGL, Inge, 8010 Graz (AT); ALLAM, Menna Allah, 8055 Graz (AT); STEFFAN, Christoph, 8042 Graz (AT)
(74) Representative: Westphal, Mussgnug & Partner, Patentanwälte mbB

(56) References cited:
- US-A1- 2003 206 021
- US-A1- 2017 184 539
- US-A1- 2018 321 187

## Description

### TECHNICAL FIELD

The present description relates to the field of electrochemical measurement techniques, and more particularly to the validation of voltammetric measurements, which may be made using a potentiostat.

### BACKGROUND

The trend to monitor various health and fitness parameters is driving the development of biochemical sensors for the consumer market as well as medical engineering products. For this market, it is important to perform the necessary measurements using low-cost electronic components that can be fabricated using, for example, standard CMOS technology. Such electronic components typically operate with a supply voltage range (rail-to-rail) of, for example, 3 V or less.

Among the measurement techniques mentioned are amperometry and voltammetry, such as cyclic voltammetry, performed using, for example, potentiostats. For example, such measurement techniques can be used to determine the concentrations of certain metal ions in a solution. One application example is the measurement of the glucose concentration in a patient's blood. Commercially available potentiostats or similar devices are usually too expensive for the consumer market and use comparatively complex electronic components that have a relatively wide voltage range available (e.g., 6 V) compared to the above-mentioned value of 3 V, which cannot be readily provided by modern standard CMOS components, but may be necessary due to the electrochemical properties of the materials to be analyzed. Reference is made to US20180321187A1 which describes a sensor designed for cyclic voltammetry.

Measurement set-ups including potentiostats for voltammetric measurements typically excite the electrochemical cell in a fixed way, for example, by setting the voltage of a reference electrode of the electrochemical cell to a constant value (which may be zero), which may be optimized for a given supply voltage range and applying a desired voltage at a working electrode of the electrochemical cell. The resulting cell current depends on the concentration of an analyte present in the electrolyte of the electrochemical cell. However, parasitic passive components (e.g. capacitors and resistors), which cannot be fully avoided in practice, may have an undesired impact on the resulting cell current and thus may deteriorate the measured data. As the mentioned parasitic components are a priori unknown, an object of the present invention may be seen as the improvement of the reliability of the measurement process although unavoidable parasitic components are present.

### SUMMARY

The mentioned objective is achieved by the method of claim 1. Various embodiments and further developments are covered by the dependent claims.

In accordance with one embodiment, the method includes exciting an electrochemical cell, which includes a working electrode, a reference electrode and a counter electrode, in accordance with a selected excitation mode of two or more different excitation modes such that a cell voltage between the working electrode and the reference electrode tracks a desired cell voltage. The method further includes repeatedly measuring a cell current (current response) while exciting the electrochemical cell with different selected excitation modes. A consistency check is performed based on the measured cell currents associated with the different excitation modes.

Another embodiment relates to a measurement set-up which may be used for cyclic voltammetry. In accordance with one embodiment, the measurement set-up includes an electrochemical cell with a working electrode, a reference electrode, and a counter electrode for contacting the electrochemical cell, and a circuit arrangement (e.g. a potentiostat) coupled to the electrochemical cell. The circuit arrangement is configured to excite the electrochemical cell in accordance with a selected excitation mode of two or more different excitation modes such that a cell voltage between the working electrode and the reference electrode tracks a desired cell voltage. The circuit arrangement is further configured to repeatedly measure a cell current while the electrochemical cell is excited using different selected excitation modes and to perform a consistency check based on the measured cell currents associated with the different excitation modes.

### BRIEF DESCRIPTION OF THE DRAWINGS

The invention can be better understood with reference to the following drawings and descriptions. The components in the figures are not necessarily to scale; instead emphasis is placed upon illustrating the principles of the invention. Moreover, in the figures, like reference numerals designate corresponding parts. In the drawings:
Figure 1 is a schematic of an example of an interface circuit for operating an electrochemical cell suitable for voltammetric analysis techniques.
Figure 2 illustrates a block diagram of a system for performing voltammetry and related analysis techniques.
Figure 3 shows in a diagram the signal waveforms at the electrodes of a cell during cyclic voltammetry.
Figure 4 illustrates an example similar to the circuit of Fig. 1 with parasitic capacitances coupled to the electrochemical cell.
Figure 5 illustrates an example of the electrical excitation of an electrochemical cell in three different modes.
Figure 6 illustrates cell current over cell voltage for the measurements shown in Fig. 5.
Figure 7 is a flow chart illustrating an exemplary embodiment of the method described herein.

### DETAILED DESCRIPTION

Cyclic voltammetry (CV) is a measurement and analysis technique commonly used in the field of electrochemistry for the qualitative and quantitative analysis of a sample, by means of which the chemical composition of mixtures of substances can be determined on the basis of the voltage-dependent current curve (waveform). Voltammetry is a form of electrolysis in which the dependence of an electrode current on a voltage applied to an electrochemical cell (cell voltage) is determined. Further examination of the sample involves evaluating the measured current-voltage curves, for example, to determine the concentration of an analyte (e.g., certain metal ions) contained in the sample.

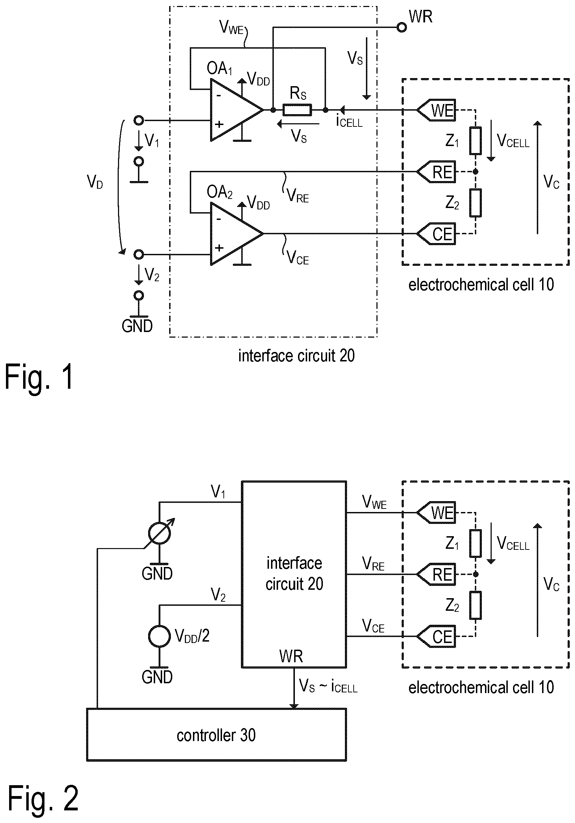

Fig. 1 is a schematic of an example of a circuit for operating an electrochemical cell which is suitable for voltammetric analysis techniques. The circuit of Fig. 1 includes an electrochemical system, referred to herein as electrochemical cell 10. In the depicted example, the cell 10 comprises three electrodes located in an electrolyte (i.e. the sample to be analyzed). These electrodes are commonly referred to as the working electrode (WE), the reference electrode (RE), and the counter electrode (CE), with the counter electrode sometimes also being referred to as the auxiliary electrode. In an electric model, the current path between the working electrode WE and the reference electrode RE can be considered as an impedance Z₁, and the current path between the reference electrode RE and the counter electrode CE can be considered as an impedance Z₂. These impedances Z₁ and Z₂ depend, inter alia, on the applied cell voltage V_{CELL}, the chemical properties of the electrolyte present in the cell 10, and the chemical reactions taking place in the cell. In the example shown in Fig. 1, the cell 10 is an electrical load represented by the impedances Z₁ and Z₂. Depending on the electrolyte, the cell 10 may also be a galvanic cell operating as a voltage source (with internal resistance).

In voltammetry, the cell voltage V_{CELL} between the working electrode WE and the reference electrode RE is usually actively adjusted according to a set-point, which may change over time. In order to control the cell voltage V_{CELL} sufficiently well, it is usually necessary in practice that the reference electrode RE remains currentless, which means that no significant current flows across the reference electrode RE so that redox reactions at the reference electrode RE are avoided. The counter electrode CE is required to complete the circuit. The current i_{CELL}, which passes through the cell 10, then flows only via the working electrode WE and the counter electrode CE. The cell voltage V_{CELL} used for a specific measurement may be specified by the user. The voltage that has to be applied between the counter electrode CE and the working electrode WE to achieve the desired cell voltage V_{CELL} between the working electrode WE and the (currentless) reference electrode RE is called the compliance voltage Vc. The required compliance voltage V_{C} can be significantly higher than the cell voltage V_{CELL}, especially if the resistance of the electrolyte (impedances Z₁, Z₂, wherein Z₂ is usually much higher than Z₁) is high. As mentioned, the cell voltage V_{CELL} may be variable with time. In cyclic voltammetry, the cell voltage V_{CELL} has a periodic AC component, whereas in amperometry the cell voltage is constant.

The interface circuit 20 shown in Fig. 1 illustrates an example of how the cell voltage V_{CELL} can be adjusted. The circuit 20 may be considered as an interface between the cell 10 and a higher-level evaluation unit (not shown in Fig. 1). The electrode voltages at the working electrode WE, the reference electrode RE and the counter electrode CE are denoted in the following as V_{WE}, V_{RE} and VCE, respectively, where V_{CELL}=V_{WE}-V_{RE} and V_{C}=V_{CE}-V_{WE}. In the present example, the voltage V_{WE} is provided - based on a first input voltage V₁ - at the working electrode WE by means of a first amplifier circuit. The amplifier circuit may include an operational amplifier OA₁, which is connected as a buffer amplifier, i.e. the first amplifier circuit has a gain of one (i.e. V_{WE} = V₁). The input voltage V₁ is applied to the non-inverting input of the operational amplifier OA₁, and the output of the operational amplifier OA₁ is connected, via the sense resistor R_{S}, to the inverting input of the operational amplifier OA₁ and to the working electrode WE. The sense resistor R_{S} is just one of a number of ways to measure the output current (which is equal to the current i_{CELL} through cell 10) of the operational amplifier OA₁. In the depicted example shown, the operational amplifier OA₁ produces an output voltage of V₁-V_{S} = V_{WE}-R_{S}·i_{CELL}. The voltage across the sense resistor V_{S} = R_{S}·i_{CELL} (and thus the current i_{CELL}) can be determined, for example, by means of a differential amplifier (not shown) whose output voltage is proportional to the cell current i_{CELL.} In the depicted example, the output voltage of the operational amplifier OA1 is provided at an output node WR. The voltage V_{WR} at the output node WR is equal to V_{WE}-R_{S}·i_{CELL} and thus the voltage difference V_{WE}-V_{WR} represents the cell current i_{CELL.}

The voltage V_{RE} at the reference electrode is adjusted by means of a second amplifier circuit, the output of which is connected to the counter electrode CE and which can adjust the voltage V_{CE} at the counter electrode CE such that a predetermined voltage V₂ is present at the reference electrode RE (V_{RE} = V₂). According to the depicted example, the second amplifier circuit comprises an operational amplifier OA₂. The voltage V₂ is supplied to the non-inverting input of the operational amplifier OA₂ as an input voltage. The output of the operational amplifier OA₂ is connected to the inverting input of the operational amplifier OA₂ via the cell 10 (more precisely via the impedance Z₂). Due to the feedback loop, the operational amplifier OA₂ generates, as output voltage, the voltage V_{CE} at the counter electrode CE in such a way that V_{RE} = V₂ applies with sufficient accuracy.

In Fig. 1, it can be seen that the voltage V_{RE} at the reference electrode can be set directly (the electrode voltage V_{RE} is equal to the input voltage V₂). In known measurement systems, V_{RE} is usually set to a constant value. Furthermore, the cell voltage V_{CELL} can also be set directly, because the cell voltage V_{CELL} is equal to the difference V₁-V₂ of the input voltages V₁ and V₂. For example, if the input voltage V₂ is zero volts (ground potential), then the electrode voltage V_{RE} is constantly zero volts and the cell voltage V_{CELL} is equal to the input voltage V₁ (V_{RE} = V₂ = 0V, V_{CELL} = V₁). In this situation, a bipolar supply of the operational amplifiers OA₁ and OA₂ is usually required. In some applications with unipolar supply of the operational amplifiers OA₁ and OA₂ (supply voltage V_{DD}), the reference electrode CE can be placed, for example, at half the supply voltage (i.e. V₂ = V_{DD}/2). In this case, a maximum value of V_{DD}/2+|V_{CELL}| remains for the compliance voltage Vc, which may be too little for some applications, if the supply voltage V_{DD} is in a range of, e.g., 2-3 V, which is common for modern CMOS technologies. "Too little" in this case means that the maximum possible compliance voltage Vc is not sufficient to set the cell voltage V_{CELL} at the desired value.

It is understood that the interface circuit 20 shown in Fig. 1 is only one possible implementation, and one skilled in the art would be able to implement the same functionality in a different way. For example, the electrode voltage V_{RE} at the reference electrode RE could be measured by means of an analog-to-digital converter (ADC), and the voltages V_{WE} and V_{CE} at the working and counter electrodes WE, CE could be generated by means of digital-to-analog converters (DACs). In this example, the setting of the voltages can be done digitally, e.g., by means of a programmable microcontroller. The microcontroller may adjust the digital input signals of the DACs such that the measured value for the electrode voltage V_{RE} sensed by the ADC as well as the cell voltage V_{CELL} follow the desired value.

Fig. 2 shows a block diagram of a system for performing voltammetry and related analysis methods with an electrochemical cell 10, an interface circuit 20 (see e.g. Fig. 1) and a control unit 30 (controller), which is configured to control the measurement process and to evaluate the resulting signals. The specific implementation of the interface circuit (e.g. analog according to Fig. 1 or a partially digital implementation) is not further relevant for the further discussion. In the present example, the electrochemical cell 10 is constructed in the same way as in the example of Fig. 1. Usually, the interface circuit 20 is configured so that the voltage V_{RE} of the reference electrode RE is at half the supply voltage V_{DD} (V_{RE} = V₂ = V_{DD}/2). In the case of a bipolar, balanced supply voltage, the voltage V_{RE} may be set to 0 volts. However, the voltage V_{RE} may also be set to different voltage values.

In the illustrated example, the control unit 30 is configured to vary the voltage V₁ according to a desired signal waveform. The interface circuit 20 generates therefrom the voltage V_{WE} of the working electrode WE and the voltage V_{CE} of the counter electrode CE, the voltage V_{CE} being set such that the voltage V_{WE} of the working electrode WE follows the (variable) input voltage V₁, while the voltage V_{RE} at the currentless reference electrode RE remains substantially constant (at V_{DD}/2 in the depicted example). The control unit 30 receives, from the interface circuit 20, the measured voltage at circuit node WR representing the electrode current i_{CELL.} It should be noted at this point that the voltage V_{RE} of the reference electrode RE can also be varied according to a desired signal waveform, while the working electrode WE is at constant voltage V_{WE} (e.g. V_{WE} = V_{DD}/2). Moreover, both voltages V_{RE} and V_{WE} may vary while being adjusted such that the cell voltage corresponds to the desired (time-dependent) set-point.

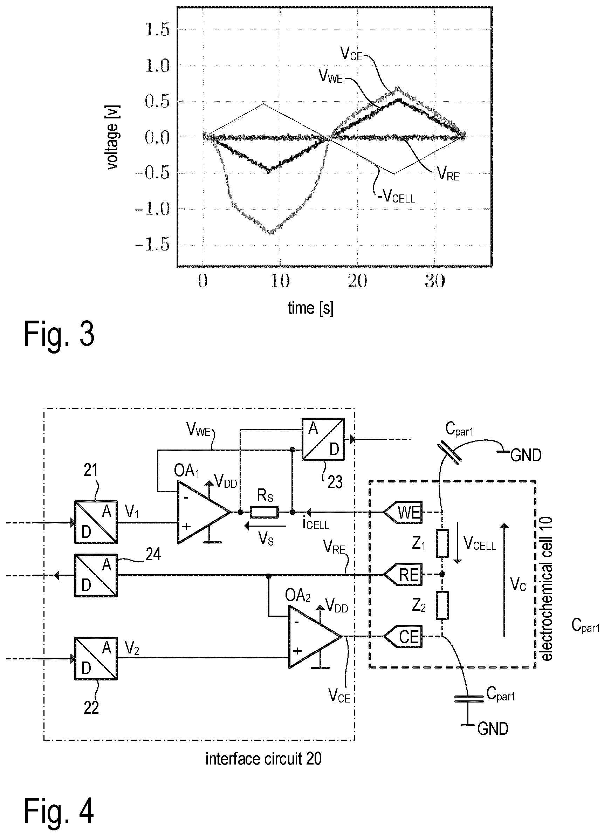

Fig. 3 illustrates, in a diagram, exemplary signal waveforms at the electrodes of cell 10 during cyclic voltammetry. According to Fig. 3, the voltage V_{WE} of the working electrode WE is periodically varied according to a triangular signal, while the reference electrode RE is at constant voltage V_{RE}. The resulting voltage V_{CE} of the counter electrode CE and the electrode current i_{CELL} (not shown in Fig. 3) can be considered as measurement signals, which can subsequently be evaluated to determine the sought properties of the electrolyte in the cell 10.

In the example shown in Fig. 3, the interface circuit 20 is operated with a bipolar supply voltage (e.g., +/-3V). The maximum value of the periodically varying cell voltage V_{CELL} (V_{CELL} = V_{WE}-V_{RE}) is 0.5 volts. The necessary (to keep the reference voltage constant at approx. 0V) voltage V_{CE} of the counter electrode reaches a minimum value of approx. -1.3 volts. In this case a voltage range of 2 V between minimum and maximum value is covered by the available range of the voltage supply.

As mentioned, the device illustrated in Fig. 2 is called a potentiostat, which can be used for (cyclic) voltammetry. For this purpose, a voltage signal (cell voltage V_{CELL}) is applied to the electrochemical cell to measure the resulting cell current i_{CELL} at the working electrode. Ideally, the current i_{CELL} depends only on the properties of electrochemical cell and is independent from the surrounding environment. However, in practice, parasitic electric components (e.g. parasitic resistors or capacitors) cannot be avoided, and their presence may cause an error in the voltammetric measurements. In voltammetry, the resulting current-voltage curves (e.g. cell current over cell voltage) may be evaluated to detect a specific analyt in the cell. However, these curves may be altered by the mentioned parasitic electric components, wherein it may be difficult for a user of the potentiostat to recognize whether a measured curve actually represents the properties of the electrochemical cell or has been altered/deteriorated by parasitic electric components. The concepts described herein aim at alleviating this problem.

Current approaches try to avoid parasitic components as good as possible, but there is no easy-to-use mechanism for the validation of voltammetric measurements. For example, to reduce undesired displacement currents caused by the charging of parasitic capacitances, many known potentiostats are configured to keep the working electrode at a constant electric potential during measurements. This, however, does not fully solve the problem, because the cell current i_{CELL} measured at the working electrode can, nevertheless, have a current component due to the charging of a parasitic capacitance at the reference electrode. Furthermore, a constant potential of the working electrode only helps to reduce displacement currents due to the charging and discharging of parasitic capacitances, but parasitic (ohmic) resistances may still cause leakage currents that may contribute to a systematic error in the "true" cell current. Moreover, keeping the working electrode at a constant potential is undesirable in systems with a relatively small supply voltage.

The embodiments described herein address the problems described above by performing the same measurement multiple times using different electrical excitation of the electrodes WE, RE, and CE. For example, the controller 30 and/or the interface circuit 20 (see Fig. 2) of a potentiostat is configured to operate in different modes, which differ in the electrical excitation of the cell electrodes WE, RE, and CE. The resulting cell voltage V_{CELL} is the same in all modes and, therefore, the same measurement is performed in each (excitation) mode. However, due to the different electrical excitation of the electrodes WE, RE, and CE, the parasitic electrical components may behave differently (and thus differently affect the measured cell current i_{CELL}) in the different modes.

By performing the same measurement multiple times using different excitation modes allows to check the validity of the measurement setup by checking the consistency of the multiple measurements.

As mentioned above, the cell voltage V_{CELL} is given by the equation V_{CELL}=V_{WE}-V_{RE}, wherein the voltage V_{WE} equals V₁ and the voltage V_{RE} equals V₂ in the examples of Fig. 1 and 2, accordingly ${V}_{CELL} = {V}_{WE} - {V}_{RE} = {V}_{1} - {V}_{2} .$ For the further discussion, it is assumed that the desired cell voltage V_{CELL} is given by the periodic signal V_{CELL}*(t), which can be regarded as the time-variant set-point for the cell voltage V_{CELL}. It follows from the above equation, that the electrical excitations ${V}_{1} = {V}_{CELL} * \left(t\right) and {V}_{2} = 0 V \left(first mode\right) ,$ and ${V}_{1} = 2.5 V and {V}_{2} = 2.5 V - {V}_{CELL} * \left(t\right) \left(second mode\right) ,$ will result in exactly the same cell voltage V_{CELL} and, if no parasitic electric components are present, in exactly the same current sense signal V_{S} (representing the cell current i_{CELL}, see Fig. 2).

It is understood that the excitation modes, which are labelled "first mode" and "second mode" are merely an example, and many more modes may be implemented. In one embodiment, for example, both voltages V₁ and/or V₂ are varied, in one mode, such that the electric potential of the counter electrode CE remains substantially constant. In a further mode, the voltages at all three electrodes may vary (basically in an arbitrary way) provided that the voltage difference V_{WE}-V_{RE} equals the desired signal V_{CELL}*(t).

In absence of parasitic electric components in the electrochemical system, the cell voltage V_{CELL} and the measured cell current i_{CELL} are identical for all excitation modes, but there will be differences when parasitic components are present. If the influence of the parasitic components is small, the measured curves (e.g. cell current over cell voltage) will be at least approximately the same (within certain tolerance ranges). However, if the parasitic components have a significant impact on the current measurement, then the measurements made using different excitation modes will become inconsistent. Hence, a consistency check may help to identify unreliable measurements.

Fig. 4 illustrates an example similar to the circuit of Fig. 1 with examples of the parasitic capacitances coupled to the electrochemical cell as discussed above. The structure of the electrochemical cell 10 is basically the same as in Fig. 1 and reference is made to the description above. The only difference between the example of Fig. 4 and the circuit of Fig. 1 is that the voltages V₁ (=V_{WE}) and V₂ (=V_{RE}) are generated by digital-to-analog converters (DACs) denoted as DAC 21 and 22, respectively, in Fig. 4. Further, the current sense signal i_{CELL}·R_{S} that represents the cell current as well as the voltage V_{RE} at the reference electrode RE are measured using analog-to-digital converter (ADCs) denoted as ADC 23 and 24, respectively.

Also shown in Fig. 4 are the parasitic capacitances Cₚₐᵣ₁ and Cₚₐᵣ₂ coupled to the working electrode WE and the counter electrode CE. A digital controller (not shown in Fig. 4), e.g. a microcontroller or the like, which includes memory and a processor for executing software instructions, may be used to read the digital output signals of the ADCs 23 and 24 and to generate the digital input signals for the DACs 21 and 22. To achieve the waveforms shown in Fig. 2, the digital controller may generate (using the DACs 21 and 22) - in a specific excitation mode - the voltage V₁ and V₂ such that the voltage V_{RE} at the reference electrode RE is substantially constant and zero (i.e. V_{RE}=V₂ ≈ 0V) and that the voltage V_{WE} at the working electrode WE is substantially equal to the desired cell voltage V_{CELL}*(t) (i.e. V₁ = V_{CELL}*(t)).

It is noted that, with a small modification / reconfiguration the interface circuit 20 of Fig. 4 may be configured to keep the voltage V_{CE} constant (or at a time-dependent set-point value). For this purpose, the inverting input of operational amplifier OA₂ needs to be connected to the counter electrode CE instead of the reference electrode RE. The voltage V₂ will then directly determine the voltage V_{CE} (i.e. V_{CE}=V₂) while the voltage V_{RE} is floating and measured by ADC 24. Such a reconfiguration may be accomplished automatically, e.g. by using electronic switches, and the mentioned controller may trigger such a reconfiguration]. In this example (constant V_{CE}), the output of ADC 24 is used to regulate the voltage V₁ such that the cell voltage V_{CELL} tracks the desired cell voltage V_{CELL}*(t).

With a suitable interface circuit (in combination with a controller 30, as the case may be, see also Fig. 2), the electrochemical cell can be electrically excited in various excitations modes. In order to be able to perform a consistency check, a specific measurement with a specific (desired) cell voltage V_{CELL}(t) is repeated using two or more excitation modes.

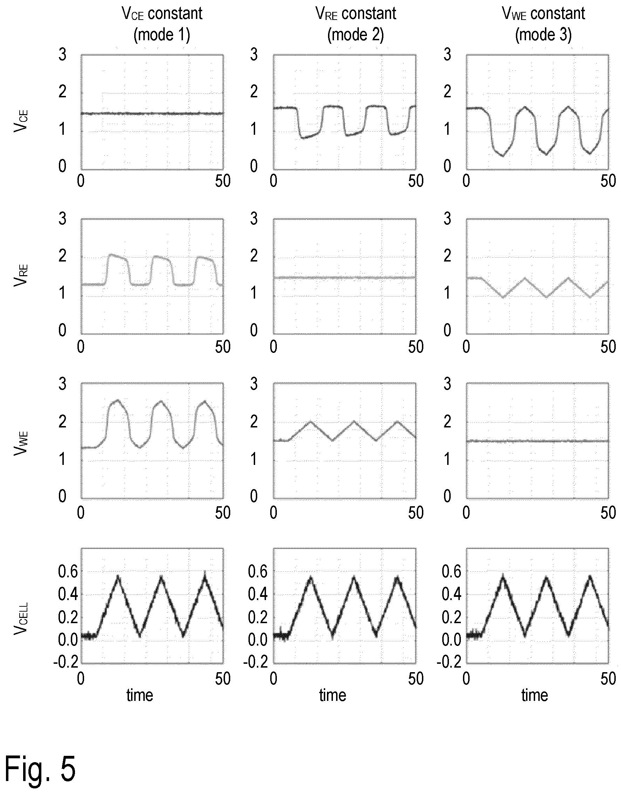

The timing diagrams of Fig. 5 illustrate the electric excitation of the electrochemical cell 10 in three different excitation modes labelled "mode 1", "mode 2", and "mode 3". The timing diagrams in the first (from top to bottom) line illustrate the voltage V_{CE} at the counter electrode CE, the timing diagrams in the second line illustrate the voltage V_{RE} at the reference electrode WE, the timing diagrams in the third line illustrate the voltage V_{WE} at the working electrode, and the timing diagrams in the fourth line illustrate the resulting voltage V_{CELL}(t), which is the difference between V_{WE} and V_{RE}. The reference electrode RE is currentless as shown in the examples of Figs. 1 and 4, wherein currentless means the electrode current is substantially zero except the negligible input voltages of the operational amplifier OA₂ and the ADCs.

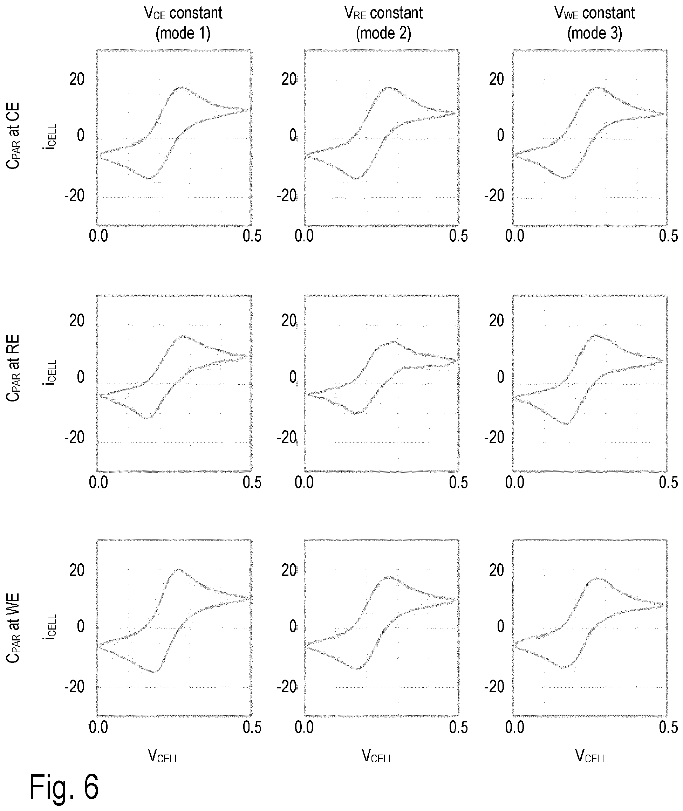

It can be seen from Fig. 5 that the resulting cell voltages V_{CELL}(t) are substantially equal in the difference modes. Fig. 6 shows nine diagrams, three for each mode, illustrating cell current i_{CELL} over cell voltage V_{CELL} corresponding to the measurements of Fig. 5, wherein the diagrams in the left column correspond to excitation mode 1 (V_{CE} constant), the diagrams in the middle column correspond to excitation mode 2 (V_{RE} constant), and the diagrams in the left column correspond to excitation mode 3 (V_{WE} constant). The measurements illustrated in the diagrams of the first line (from top to bottom) have been made with a parasitic capacitance at the counter electrode CE. The measurements illustrated in the diagrams of the second line have been made with a parasitic capacitance at the reference electrode RE, and the measurements illustrated in the diagrams of the third line have been made with a parasitic capacitance at the working electrode WE. As the parasitic capacitances are not very large, the resulting V_{CELL}/i_{CELL}-diagrams are rather similar in the different excitation modes although not identical.

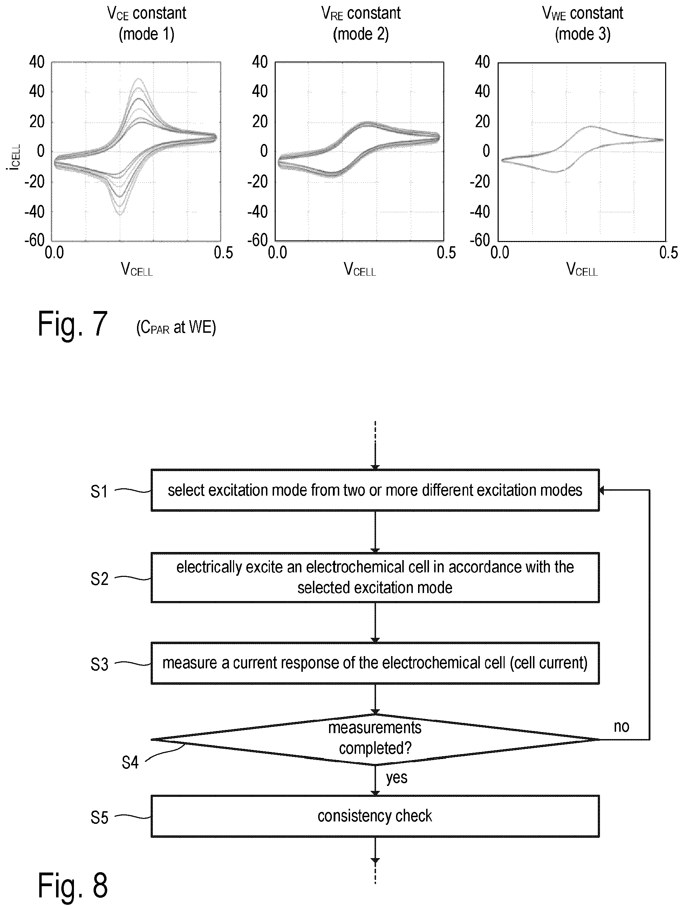

Fig. 7 includes three different V_{CELL}/i_{CELL}-diagrams, wherein the underlying data has been measured using different excitation modes (left diagram corresponds to excitation mode 1 with constant V_{CE}, middle diagram corresponds to excitation mode 2 with constant V_{RE}, and right diagram corresponds to excitation mode 3 with constant V_{WE}). Each diagram contains several curves that have been measured with different parasitic capacitances coupled to the working electrode WE. One can see that the resulting V_{CELL}/i_{CELL}-curves significantly depend on the value of the parasitic capacitances except in exciting mode 3, in which the potential V_{WE} at the working electrode WE is constant. Similar diagrams can be obtained for parasitic capacitances coupled to the reference electrode RE and the counter electrode CE.

The parasitic capacitances are difficult to control and may be different in each measurement setup and also vary over time, temperature, etc. As discussed with reference to Fig. 5 and 6, the impact of the parasitic capacitances may be negligibly as long as the sizes of the parasitic components are small. As mentioned a consistency check may be performed when the measurement is repeated in different excitation modes. The approach is summarized below with reference to the flow chart shown in Fig. 7.

In accordance with the embodiment of Fig. 7, the method includes the selection of an excitation mode from two or more different excitation modes (see Fig. 7, step S1) and exciting an electrochemical cell (cf. Fig. 2, electrochemical cell 20 including a working electrode WE, a reference electrode RE and a counter electrode CE) in accordance with the selected excitation mode such that a resulting cell voltage V_{CELL} between the working electrode WE and the reference electrode RE tracks a desired cell voltage V_{CELL}*(t) (see Fig. 7, step S2). The cell voltage V_{CELL} tracking the desired cell voltage V_{CELL}*(t) means that the voltages applied at the working electrode WE, the reference electrode RE and the counter electrode CE are adjusted such that the cell voltage V_{CELL} is substantially equal to the desired cell voltage V_{CELL}*(t) within a given range of tolerance.

The method further includes measuring a current response of the electrochemical cell, i.e. the cell current i_{CELL}, while exciting the electrochemical cell with the selected excitation mode (see Fig. 7, step S3). The steps S1, S2, and S3 are repeated (at least one time, see Fig. 7, step S4) until each available excitation mode (or at least two different modes thereof) has been selected. After the measurements have been completed, a consistency check is performed (see Fig. 7, step S5) based on the measured current responses associated with the different excitation modes.

In one embodiment the consistency check may include evaluating the current responses associated with different excitation modes and indicating an inconsistency when the current responses associated with the different excitation modes differ from each other (or from a reference response) by more than a defined tolerance. In this case, the reference response may be the average of the current responses measured in different excitation modes. The evaluation of the measured current responses may include the evaluation of the measured currents-over-time curves or the measured current-over-cell voltage curves. Examples of current-over-cell voltage curves are shown in Figs. 6 and 7.

In one embodiment the excitation of the electrochemical cell may include applying a first voltage V_{WE} at the working electrode WE, a second voltage V_{RE} at the reference electrode RE and a third voltage V_{CE} at the counter electrode CE, such that the cell voltage V_{CELL} (equals V_{WE}-V_{RE}) tracks the desired cell voltage V_{CELL}*(t). Dependent on the excitation mode, a specific one of the voltages V_{WE}, V_{RE}, V_{CE} is set to a defined set-point value, which may be constant or, in some embodiments and excitation modes, time-dependent. In the example of Fig. 1 and as discussed above, the voltage V_{RE} at the (ideally currentless) reference electrode RE is set to the set-point value defined by voltage V₂, while the other two voltages V_{WE} and V_{CE} are regulated such that the cell voltage V_{CELL} follows the desired signal V_{CELL}*(t) and the condition V_{RE}=V₂ is maintained.

In one embodiment either the working electrode WE or the reference electrode RE or the counter electrode CE is supplied with a constant voltage dependent on the excitation mode. An example is shown in Fig. 5, in which the voltage V_{CE} is constant in excitation mode 1, the voltage V_{RE} is constant in excitation mode 2 and the voltage V_{WE} is constant in excitation mode 3. Accordingly, which one of the voltages V_{WE}, V_{RE}, V_{CE} is set in accordance with a (e.g. constant) set-point value, may depend on the selected excitation mode. Further, the excitation modes may also differ by the set-point value, which may be, e.g. zero volts (or any other constant voltage level) in one mode or vary in accordance with a given time-dependent set-point in another mode. The desired cell voltage V_{CELL}*(t) may be a given periodic voltage signal. In this case, the resulting cell voltage V_{CELL} and the current responses i_{CELL} are also periodic signals.

In the embodiments described herein, the electrochemical cell includes an electrolyte and the measured cell currents (current responses) depend, inter alia, on a concentration of an analyte present in the electrolyte. In particular, the measured current response is dependent on the concentration of the redox species (the analyte) at the working electrode surface. As mentioned, the process is as such known as cyclic voltammetry and thus not further discussed herein.

Although the invention has been illustrated and described with respect to one or more implementations, alterations and/or modifications may be made to the illustrated examples without departing from the scope of the appended claims. In particular regard to the various functions performed by the above described components or structures (units, assemblies, devices, circuits, systems, etc.), the terms (including a reference to a "means") used to describe such components are intended to correspond - unless otherwise indicated - to any component or structure, which performs the specified function of the described component (e.g., that is functionally equivalent), even though not structurally equivalent to the disclosed structure, which performs the function in the herein illustrated exemplary implementations of the invention.

## Claims

1. A method comprising:
exciting an electrochemical cell (20), which includes a working electrode (WE), a reference electrode (RE) and a counter electrode (CE), in accordance with a selected excitation mode of two or more different excitation modes such that a cell voltage (V_{CELL}) between the working electrode (WE) and the reference electrode (RE) tracks a desired cell voltage (V_{CELL}*(t));
**characterised in that** the method further comprises:
repeatedly measuring a cell current (i_{CELL}) while exciting the electrochemical cell with different selected excitation modes;
performing a consistency check based on the measured cell currents (i_{CELL}) associated with the different excitation modes.

2. The method of claim 1, wherein performing the consistency check comprises:
evaluating the measured cell currents (i_{CELL}) associated with different excitation modes and
indicating an inconsistency when the measured cell currents (i_{CELL}) associated with different excitation modes differ by more than a defined tolerance.

3. The method of claim 2, wherein evaluating the measured cell currents (i_{CELL}) comprises:
evaluating the measured cell currents (i_{CELL}) over time curves or cell currents (i_{CELL}) over cell voltage (V_{CELL}) curves.

4. The method of one of claims 1 to 3, wherein exciting an electrochemical cell (20) comprises:
applying a first voltage (V_{WE}) at the working electrode (WE), a second voltage (V_{RE}) at the reference electrode (RE) and a third voltage (VCE) at the counter electrode (CE), such that the cell voltage (V_{CELL}) tracks the desired cell voltage (V_{CELL}*(t)),
wherein, dependent on the excitation mode, a specific one of the first, second or third voltage (V_{WE}, V_{RE}, V_{CE}) is set in accordance with a set-point value.

5. The method of one of claims 1 to 3,
wherein, dependent on the excitation mode, the working electrode (WE) or the reference electrode (RE) or the counter electrode (CE) is supplied with a constant voltage.

6. The method of claim 4,
wherein the set-point value depends on the selected excitation mode and/or
wherein it depends on the selected excitation mode which one of the first, second or third voltage (V_{WE}, V_{RE}, V_{CE}) is set in accordance with a set-point value.

7. The method of one of claims 1 to 6,
wherein the desired cell voltage (V_{CELL}*(t)) is a given periodic voltage signal.

8. The method of one of claims 1 to 7,
wherein the electrochemical cell (20) includes an electrolyte and the measured cell currents (i_{CELL}) depend, inter alia, on a concentration of an analyte present in the electrolyte.

9. A measurement set-up comprising:
an electrochemical cell (20) having a working electrode (WE), a reference electrode (RE) and a counter electrode (CE) for contacting the electrochemical cell (20), and
a circuit arrangement (10) coupled to the electrochemical cell (20) and configured to:
excite the electrochemical cell (20) in accordance with a selected excitation mode of two or more different excitation modes such that a cell voltage (V_{CELL}) between the working electrode (WE) and the reference electrode (RE) tracks a desired cell voltage (V_{CELL}*(t))
**characterised in that** the measurement set-up further comprises:
repeatedly measure a cell current (i_{CELL}) while the electrochemical cell is excited using different selected excitation modes, and
perform a consistency check based on the measured cell currents (i_{CELL}) associated with the different excitation modes.

10. The measurement set-up of claim 9,
wherein the circuit arrangement is included in a potentiostat.

11. The measurement set-up of claim 9 or 10, wherein, to perform the consistency check, the circuit arrangement is configured to:
evaluate the measured cell currents (i_{CELL}) associated with different excitation modes and
indicate an inconsistency when the measured cell currents (i_{CELL}) associated with different excitation modes differ by more than a defined tolerance.

12. The measurement set-up of any of claims 9 to 11, wherein to excite the electrochemical cell (20), the circuit arrangement (10) is configured to:
apply a first voltage (V_{WE}) at the working electrode (WE), a second voltage (V_{RE}) at the reference electrode (RE) and a third voltage (VCE) at the counter electrode (CE), such that the cell voltage (V_{CELL}) tracks the desired cell voltage (V_{CELL}*(t)),
wherein, dependent on the excitation mode, a specific one of the first, second or third voltages (V_{WE}, V_{RE}, V_{CE}) is set in accordance with a set-point value.

13. The measurement set-up of claim 12,
wherein the set-point value is a constant voltage value.

14. The measurement set-up of any of claims 9 to 12,
wherein the desired cell voltage (V_{CELL}*(t)) is a given periodic voltage signal.

15. The measurement set-up of any of claims 9 to 13,
wherein the electrochemical cell (20) includes an electrolyte and the measured cell currents (i_{CELL}) depend, inter alia, on a concentration of an analyte present in the electrolyte.

## Patentansprüche

1. Verfahren, umfassend:
Anregen einer elektrochemischen Zelle (20), die eine Arbeitselektrode (WE), eine Referenzelektrode (RE) und eine Gegenelektrode (CE) einschließt, gemäß einem ausgewählten Anregungsmodus von zwei oder mehr verschiedenen Anregungsmodi, so dass eine Zellspannung (V_{ZELLE}) zwischen der Arbeitselektrode (WE) und der Referenzelektrode (RE) eine gewünschte Zellspannung (V_{ZELLE}*(t)) verfolgt;
**dadurch gekennzeichnet, dass** das Verfahren ferner umfasst:
wiederholtes Messen eines Zellstroms (i_{ZELLE}), während die elektrochemische Zelle mit verschiedenen ausgewählten Anregungsmodi angeregt wird;
Durchführen einer Konsistenzprüfung basierend auf den gemessenen Zellströmen (i_{ZELLE}), die mit den verschiedenen Anregungsmodi assoziiert sind.

2. Verfahren nach Anspruch 1, wobei Durchführen der Konsistenzprüfung umfasst:
Auswerten der gemessenen Zellströme (i_{ZELLE}), die mit verschiedenen Anregungsmodi assoziiert sind, und
Angeben einer Inkonsistenz, wenn sich die gemessenen Zellströme (i_{ZELLE}), die mit verschiedenen Anregungsmodi assoziiert sind, um mehr als eine definierte Toleranz unterscheiden.

3. Verfahren nach Anspruch 2, wobei Auswerten der gemessenen Zellströme (i_{ZELLE}) umfasst:
Auswerten der gemessenen Kurven von Zellströmen (i_{ZELLE}) über die Zeit oder Kurven von Zellströmen (i_{ZELLE}) über die Zellspannung (V_{ZELLE}) .

4. Verfahren nach einem der Ansprüche 1 bis 3, wobei Anregen einer elektrochemischen Zelle (20) umfasst:
Anlegen einer ersten Spannung (V_{WE}) an der Arbeitselektrode (WE), einer zweiten Spannung (V_{RE}) an der Referenzelektrode (RE) und einer dritten Spannung (V_{CE}) an der Gegenelektrode (CE), so dass die Zellspannung (V_{ZELLE}) die gewünschten Zellspannung (V_{ZELLE}*(t)) verfolgt, wobei in Abhängigkeit von dem Anregungsmodus eine spezielle der ersten, zweiten oder dritten Spannung (V_{WE}, V_{RE}, V_{CE}) gemäß einem Sollwert eingestellt wird.

5. Verfahren nach einem der Ansprüche 1 bis 3,
wobei in Abhängigkeit von dem Anregungsmodus die Arbeitselektrode (WE) oder die Referenzelektrode (RE) oder die Gegenelektrode (CE) mit einer konstanten Spannung versorgt wird.

6. Verfahren nach Anspruch 4,
wobei der Sollwert von dem ausgewählten Anregungsmodus abhängt, und/oder
wobei es von dem ausgewählten Anregungsmodus abhängt, welche der ersten, zweiten oder dritten Spannung (V_{WE}, V_{RE}, V_{CE}) gemäß einem Sollwert eingestellt wird.

7. Verfahren nach einem der Ansprüche 1 bis 6,
wobei die gewünschte Zellspannung (V_{ZELLE}*(t)) ein gegebenes periodisches Spannungssignal ist.

8. Verfahren nach einem der Ansprüche 1 bis 7,
wobei die elektrochemische Zelle (20) einen Elektrolyten einschließt, und die gemessenen Zellströme (i_{ZELLE}) unter anderem von einer Konzentration eines in dem Elektrolyten vorhandenen Analyten abhängen.

9. Messaufbau, umfassend:
eine elektrochemische Zelle (20) mit einer Arbeitselektrode (WE), einer Referenzelektrode (RE) und einer Gegenelektrode (CE) zum Kontaktieren der elektrochemischen Zelle (20), und
eine Schaltungsanordnung (10), die mit der elektrochemischen Zelle (20) gekoppelt ist und ausgelegt ist zum:
Anregen der elektrochemischen Zelle (20) gemäß einem ausgewählten Anregungsmodus von zwei oder mehr verschiedenen Anregungsmodi derart, dass eine Zellspannung (V_{ZELLE}) zwischen der Arbeitselektrode (WE) und der Referenzelektrode (RE) eine gewünschte Zellspannung (V_{ZELLE}*(t)) verfolgt,
**dadurch gekennzeichnet, dass** der Messaufbau ferner umfasst:
wiederholtes Messen eines Zellstroms (i_{ZELLE}), während die elektrochemische Zelle unter Verwendung verschiedener ausgewählter Anregungsmoden angeregt wird, und
Durchführen einer Konsistenzprüfung basierend auf den gemessenen Zellströmen (i_{ZELLE}), die mit den verschiedenen Anregungsmodi assoziiert sind.

10. Messaufbau nach Anspruch 9,
wobei die Schaltungsanordnung in einen Potentiostaten eingeschlossen ist.

11. Messaufbau nach Anspruch 9 oder 10, wobei die Schaltungsanordnung zum Durchführen der Konsistenzprüfung ausgelegt ist zum:
Auswerten der gemessenen Zellströme (I_{ZELLE}), die mit verschiedenen Anregungsmodi assoziiert sind, und
Angeben einer Inkonsistenz, wenn sich die gemessenen Zellströme (i_{ZELLE}), die mit verschiedenen Anregungsmodi assoziiert sind, um mehr als eine definierte Toleranz unterscheiden.

12. Messaufbau nach einem der Ansprüche 9 bis 11, wobei die Schaltungsanordnung (10) zum Anregen der elektrochemischen Zelle (20) ausgelegt ist zum:
Anlegen einer ersten Spannung (V_{WE}) an der Arbeitselektrode (WE), einer zweiten Spannung (V_{RE}) an der Referenzelektrode (RE) und einer dritten Spannung (V_{CE}) an der Gegenelektrode (CE), so dass die Zellspannung (V_{ZELLE}) die gewünschten Zellspannung (V_{ZELLE}*(t)) verfolgt, wobei in Abhängigkeit von dem Anregungsmodus eine spezielle der ersten, zweiten oder dritten Spannung (V_{WE}, V_{RE}, V_{CE}) gemäß einem Sollwert eingestellt wird.

13. Messaufbau nach Anspruch 12,
wobei der Sollwert ein konstanter Spannungswert ist.

14. Messaufbau nach einem der Ansprüche 9 bis 12,
wobei die gewünschte Zellspannung (V_{ZELLE}*(t)) ein gegebenes periodisches Spannungssignal ist.

15. Messaufbau nach einem der Ansprüche 9 bis 13,
wobei die elektrochemische Zelle (20) einen Elektrolyten einschließt, und die gemessenen Zellströme (i_{ZELLE}) unter anderem von einer Konzentration eines in dem Elektrolyten vorhandenen Analyten abhängen.

## Revendications

1. Un procédé comprenant :
exciter une pile (20) électrochimique, qui comprend une électrode (WE) de travail, une électrode (RE) de référence et une contre-électrode (CE), suivant un mode d'excitation sélectionné parmi deux ou plusieurs modes d'excitation différents, de manière à ce qu'une tension (V_{CELL}) de la pile, entre l'électrode (WE) de travail et l'électrode (RE) de référence, suive une tension (V_{CELL}* (t)) souhaitée de la pile ;
**caractérisé en ce que** le procédé comprend en outre :
mesurer de manière répétée un courant (i_{CELL}) de la pile, tout en excitant la pile électrochimique avec des modes différents d'excitation sélectionnés ;
effectuer un contrôle de cohérence sur la base des courants (i_{CELL}) mesurés de la pile associés aux différents modes d'excitation.

2. Le procédé de la revendication 1, dans lequel effectuer le contrôle de cohérence comprend :
évaluer les courants (i_{CELL}) mesurés de la pile associés à différents modes d'excitation et
indiquer une incohérence, lorsque les courants (i_{CELL}) mesurés de la pile associés à des modes d'excitation différents, diffèrent de plus d'une tolérance définie.

3. Le procédé de la revendication 2, dans lequel évaluer les courants (i_{CELL}) mesurés de la pile comprend :
évaluer les courants (i_{CELL}) mesurés de la pile par des courbes en fonction du temps ou des courants (i_{CELL}) de la pile par des courbes de tension (V_{CELL}) de la pile.

4. Le procédé de l'une des revendications 1 à 3, dans lequel exciter la pile (20) électrochimique comprend :
appliquer une première tension (V_{WE}) à l'électrode (WE) de travail, une deuxième tension (V_{RE}) à l'électrode (RE) de référence et une troisième tension (VCE) à la contre-électrode (CE), de manière à ce que la tension (V_{CELL}) de la pile suive la tension (V_{CELL}* (t)) souhaitée de la pile,
dans lequel, en fonction du mode d'excitation, on règle une précise de la première, deuxième ou troisième tension (V_{WE}, V_{RE}, V_{CE}) suivant une valeur de point de consigne.

5. Le procédé de l'une des revendications 1 à 3,
dans lequel, en fonction du mode d'excitation, l'électrode (WE) de travail ou l'électrode (RE) de référence ou la contre-électrode (CE) est alimentée en une tension constante.

6. Le procédé de la revendication 4,
dans lequel la valeur du point de consigne dépend du mode d'excitation sélectionné et/ou
dans lequel elle dépend du mode d'excitation sélectionné suivant lequel l'une de la première, deuxième ou troisième tension (V_{WE}, V_{RE}, V_{CE}) est réglée en fonction d'un point de consigne.

7. Le procédé de l'une des revendications 1 à 6,
dans lequel la tension (V_{CELL}* (t)) souhaitée de la pile est un signal de tension périodique donné.

8. Le procédé de l'une des revendications 1 à 7,
dans lequel la pile (20) électrochimique comprend un électrolyte et le courant (i_{CELL}) mesuré de la pile dépend entre autres de la concentration d'un analyte présent dans l'électrolyte.

9. Un montage de mesure comprenant :
une pile (20) électrochimique ayant une électrode (WE) de travail et une électrode (RE) de référence et une contre-électrode (CE) pour la mise en contact de la pile (20) électrochimique et
un agencement (10) de circuit connecté à la pile (20) électrochimique et configuré pour :
exciter la pile (20) électrochimique suivant un mode d'excitation sélectionné parmi deux ou plusieurs modes d'excitation différents, de manière à ce qu'une tension (V_{CELL}) de la pile entre l'électrode (WE) de travail et l'électrode (RE) de référence suive une tension (V_{CELL}*(t)) souhaitée de la pile,
**caractérisé en ce que** le montage de mesure comprend en outre :
mesurer de manière répétée un courant (i_{CELL}) de la pile tout en excitant la pile électrochimique avec des modes différents d'excitation sélectionnés, et
effectuer un contrôle de cohérence sur la base des courants (i_{CELL}) mesurés de la pile associés aux différents modes d'excitation.

10. Le montage de mesure de la revendication 9,
dans lequel l'agencement de circuit est inclus dans un potentiostat.

11. Le montage de mesure de la revendication 9 ou 10, dans lequel, pour effectuer le contrôle de cohérence, l'agencement de circuit est configuré pour :
évaluer les courants (i_{CELL}) mesurés de la pile associés à différents modes d'excitation et
indiquer une incohérence, lorsque les courants (i_{CELL}) mesurés de la pile associés à des modes d'excitation différents, diffèrent de plus d'une tolérance définie.

12. Le montage de mesure de l'une quelconque des revendications 9 à 11, dans lequel pour exciter la pile (20) électrochimique, l'agencement (10) de circuit est configuré pour :
appliquer une première tension (V_{WE}) à l'électrode (WE) de travail, une deuxième tension (V_{RE}) à l'électrode (RE) de référence et une troisième tension (VCE) à la contre-électrode (CE), de manière à ce que la tension (V_{CELL}) de la pile suive la tension (V_{CELL}* (t)) souhaitée de la pile,
dans lequel, en fonction du mode d'excitation, on règle une précise de la première, deuxième ou troisième tension (V_{WE}, V_{RE}, V_{CE}) suivant une valeur de point de consigne.

13. Le montage de mesure de la revendication 12,
dans lequel la valeur du point de consigne est une valeur constante de la tension.

14. Le montage de mesure suivant l'une quelconque des revendications 9 à 12,
dans lequel la tension (V_{CELL}* (t)) souhaitée de la pile est un signal de tension périodique donné.

15. Le montage de mesure de l'une quelconque des revendications 9 à 13,
dans lequel la pile (20) électrochimique comprend un électrolyte et le courant (i_{CELL}) mesuré de la pile dépend entre autres d'une concentration d'un analyte présent dans l'électrolyte.
